# EUROPEAN PATENT SPECIFICATION

(11) **EP 2 571 553 B1**
(45) Date of publication and mention of the grant of the patent: **06.08.2014**
(21) Application number: 11721494.0
(22) Date of filing: 17.05.2011
(51) Int. Cl.: A61M 5/32, A61M 5/24, A61M 5/34

(54) **NEEDLE ASSEMBLY FOR DRUG DELIVERY DEVICES**
NADELANORDNUNG FÜR ARZNEIMITTELVERABREICHUNGSVORRICHTUNGEN
ENSEMBLE FORMANT UNE AIGUILLE POUR DISPOSITIFS D'ADMINISTRATION DE MÉDICAMENTS

(30) Priority: 18.05.2010 EP 10163047
(43) Date of publication of application: 27.03.2013
(73) Proprietor: Sanofi-Aventis Deutschland GmbH, 65929 Frankfurt am Main (DE)
(72) Inventor: JUGL, Michael, 65926 Frankfurt am Main (DE); TEUCHER, Axel, 65926 Frankfurt am Main (DE)
(86) International application number: PCT/EP2011/057957
(87) International publication number: WO 2011/144604

(56) References cited:
- WO-A1-2007/130061
- WO-A1-2008/059063
- WO-A1-2010/023488
- GB-A- 2 202 747
- US-A- 2 828 743
- US-A1- 2009 163 859

## Description

The present invention relates to a drug delivery device and in particular to a needle assembly to be interconnected to a cartridge holding section of a drug delivery device.

Drug delivery devices allowing for multiple dosing of a required dosage of a liquid medicament, such as liquid drugs, and further providing administration of the liquid to a patient, are as such well-known in the art. Generally, such devices have substantially the same purpose as that of an ordinary syringe.

Such drug delivery devices have to meet a number of user specific requirements. For instance in case of those with diabetes, many users will be physically infirm and may also have impaired vision. Therefore, these devices need to be robust in construction, yet easy to use, both in terms of the manipulation of the parts and understanding by a user of its operation. Further, the dose setting must be easy and unambiguous and where the device is to be disposable rather than reusable, the device should be inexpensive to manufacture and easy to dispose.

In order to meet these requirements, the number of parts and steps required to assemble the device and an overall number of material types the device is made from have to be kept to a minimum.

Typically, the medicament to be administered is provided in a cartridge that has a moveable piston or bung mechanically interacting with a piston rod of a drive mechanism of the drug delivery device. By applying thrust to the piston in distal direction, a certain amount of the medicinal fluid can be expelled from the cartridge.

Drug delivery devices, such like pen-type injectors typically comprise a housing having a cartridge holder for receiving the cartridge filled with the medicament that has to be dispensed. The distal end section of a cartridge holder facing towards the patient during an injector procedure typically comprises a through opening that provides access to a sealed distal end of the cartridge. By way of said through opening, an injection needle or cannula may penetrate the elastic seal to establish a fluid interconnect allowing the medicament to be expelled from the cartridge.

Typically, the disposable injection needle is provided by way of a needle assembly for releasably fastening the injection needle to the cartridge holder. For this purpose, needle assembly and distal end section of the cartridge holder comprise mutually corresponding threads, by way of which the needle assembly is screwed onto the cartridge holder. However, a threaded connection of cartridge holder and needle mount comes along with a number of deficiencies. Such a threaded engagement for instance, does not give a perceptible feedback to the user whether the needle assembly is securely mounted on the cartridge holder. In practical use, situations may occur, that only a single or a few threads of a cartridge holder and a needle assembly inter-engage, but the needle assembly is not yet securely fastened to the cartridge holder.

A user may then simply not be not aware of such improper and insufficient fastening and may therefore initiate a dose selecting and dispensing procedure. In particular in the course of a dispensing of a dose of the medicament, the needle assembly may autonomously disengage from the cartridge holder, for instance due to a built-up of a fluid pressure and/or when penetrating the skin of the patient. In these cases, the patient would be exposed to an increased health risk.

Document US 2009/0163859 A1 shows a needle assembly, e.g. Fig.9 and Fig.10, according to the preamble of claim 1.

Document WO 2010/023488 A1 describes a needle assembly for an injection pen, that has a sleeve supporting internally a double-ended needle having an injection end and a non-injection end. The end of the sleeve surrounding the non-injection end of the needle is divided into deformable segments and is internally threaded for inter engagement with a threaded boss of the injection pen. A collar is mounted on the sleeve for sliding movement between a first position where the collar overlies the segmented end and maintains inter engagement of the threads at the end with those of the injection pen boss and a second section where the segments may expand radially to come free of the injection pen boss.

It is therefore an object of the present invention, which is defined in claim 1, to provide an improved fastening mechanism for a needle assembly and to provide an unambiguous mutual mechanical engagement of cartridge holder and needle assembly. In another object, the invention also aims to provide an easy and user-friendly way of releasably fastening a needle mount to a cartridge holder of a pen-type medical injection device. It is a further object, to provide a needle assembly mount which is beneficial with respect to production costs and which is particularly suitable for an industrial mass-production process.

In a first aspect, the invention provides a needle assembly for a drug delivery device, such as a pen-type injector for delivering multiple doses of a medicament, such like insulin. The drug delivery device typically comprises a housing and a drive mechanism to be operably engaged with a piston of a cartridge containing the medicament to be dispensed by the device. The housing comprises a cartridge holder or a cartridge holder section and may comprise multiple housing components, such as a main housing component adapted to accommodate the drive mechanism. The cartridge holder or the cartridge holder section is adapted to receive the cartridge.

Irrespective on whether the drug delivery device comprises a single- or multicomponent housing, the cartridge holder or cartridge holder section at its distal end is adapted to receive and to support the needle assembly according to the present invention.

The needle assembly comprises a cup-shaped receptacle having a bottom section supporting a needle element. The needle element is adapted to penetrate a pierceable seal of the cartridge which is to be positioned in the cartridge holder. The needle element typically comprises a hypodermic needle. With its tipped end facing away from the cartridge, said needle element is adapted to penetrate biological tissue and in particular the skin of a patient for administering of the medicament.

The cup-shaped receptacle of the needle assembly comprises fastening means for releasably fastening the needle assembly to the cartridge holder. The fastening means according to the present invention and/or the receptacle itself is or are convertible into a release configuration, by way of an elastic deformation of the receptacle. Hence, by elastically deforming the receptacle, its fastening means can be transformed into a release configuration, in which the needle assembly can be removed from the cartridge holder.

Preferably, the receptacle or at least sections thereof, comprise a resiliently deformable material allowing to elastically deform the receptacle. The elasticity of the material and/or the receptacle is preferably chosen in such a way, that a user is able to initiate an elastic deformation, e.g. by squeezing the sidewalls of the receptacle with his fingers.

Furthermore the fastening means is adapted to form a positive interlock with a corresponding fastening means of the cartridge holder as defined in claim 1. By way of a positive-engaged fastening of needle assembly and cartridge holder, an unambiguous fastening of the needle assembly can be provided. Needle assembly and cartridge holder are either released or inter-engaged, wherein both configurations are unambiguously recognizable by the end-user. By avoiding a gradual fastening means, such like a threaded connection of needle assembly and cartridge holder, a risk of autonomous disengaging of cartridge holder and needle assembly during dispensing of a dose can be minimized. Patient safety can therefore be improved compared to a conventional threaded engagement of needle assembly and cartridge holder.

In a further aspect, the fastening means is adapted to establish the positive interlock by displacing the cup-shaped receptacle translationally relative to the cartridge holder in a proximal direction. For mounting the needle assembly on a distal support section of the cartridge holder, the needle assembly is simply pushed onto the distal end section of the cartridge holder until mutually corresponding fastening means of needle assembly and cartridge holder inter-engage. Preferably, engagement of said fastening means is accompanied by some kind of visually and/or audibly perceivable signal, such like a clicking noise. Consequently, the user receives some kind of feedback, indicating that the needle assembly is securely fastened to the cartridge holder.

In a further preferred embodiment, the cup-shaped receptacle is oval or elliptical in cross section and comprises a resiliently deformable sidewall section. In typical application scenarios, the cup-shaped receptacle is initially oval, i.e. prior to a mounting onto the cartridge holder. In the course of mounting the needle assembly onto the cartridge holder, its cross section may become subject to modification due to mechanical interaction with the distal support section of the cartridge holder. The geometry of the cartridge holder may for instance be circular symmetric, thus enforcing or inducing the resilient deformation of the receptacle's cross section when mounted onto the cartridge holder.

In a further preferred aspect, the fastening means of the cup-shaped receptacle comprise at least two radially inwardly protruding latching elements that are arranged on opposite end points of an imaginary short axis of the ovally shaped receptacle. By having the latching elements disposed on the short side of the oval cross section of the receptacle, and by applying radially inwardly directed pressure on the long axis of the oval receptacle, the mutual distance of the latching elements can be increased. Due to such radially outwardly directed displacement, the latching elements of the receptacle and corresponding latching elements of the cartridge holder may disengage and the needle assembly can be detached and removed from the cartridge holder.

In still another embodiment, the latching elements comprise a bevelled face pointing towards a direction of assembly. When assembling needle assembly and cartridge holder, the bevelled face of the latching elements may support elastic deformation of the oval receptacle into a circular shape. Moreover, by way of the bevelled faces, a snap fit of the mutually corresponding latching elements can be attained. In effect, fastening of needle assembly and cartridge holder can be conducted in such a way, that the needle assembly is simply translationally displaced relative to the cartridge holder in proximal direction, i.e. towards the piston of the cartridge even without any twisting or turning motion.

In still another aspect the fastening means are convertible into a release configuration by applying radially inwardly directed pressure on opposite end points of an imaginary long axis of the oval receptacle. By way of exerting pressure across the oval's long axis, mutual distance between the latching elements disposed in the vicinity of the short axis can be increased for providing a decoupling and disengagement.

In a further aspect, the receptacle transforms or becomes deformed to a substantially circular symmetric cross section when converted into its release configuration. In its final assembly configuration at the cartridge holder, the receptacle returns to its substantially oval cross section while the cartridge holder is of substantially circular cross section. However, transformation of the cup-shaped receptacle into its release configuration may also be conducted in the course of assembly and may facilitate mutual engagement or snapping of corresponding fastening means of receptacle and cartridge holder.

In another independent aspect, the invention further relates to a cartridge holder subassembly for a drug delivery device. The subassembly comprises a cartridge holder adapted to receive a cartridge being filled with a medicament which is to be dispensed by the drug delivery device. The cartridge comprises a pierceable seal at a distal end portion and typically has a slidably disposed piston at the opposite, proximal end portion. By way of exerting distally directed pressure on the piston, a predefined amount of the medicament can be expelled from the cartridge if a fluid communication is established, e.g. by way of an injection needle penetrating the pierceable seal, e.g. the septum.

The cartridge holder further has a support for a needle assembly and further comprises fastening means adapted to form a positive interlock with corresponding fastening means of the above described needle assembly.

Preferably, the fastening means comprise an annular rim disposed at the outer circumference of the needle assembly support. Instead of a circumferential annular rim, it is also conceivable, that the fastening means comprise at least two or several radially extending ribs adapted to engage with radially inwardly protruding latching elements of the needle assembly.

In still another aspect, the support for the needle assembly comprises a substantially circular cross section, wherein oval or elliptical deviations from a circular cross section are still tolerable and are within the scope of the present invention. Preferably, the diameter of the circular cross section of the needle assembly support approximately equals the radial distance between latching elements of the needle assembly that are disposed at opposite sidewall sections of the receptacle, preferably overlapping with the imaginary short axis of the receptacle's oval cross section.

In a further independent aspect the invention also relates to a drug delivery device for administering at least one dose of a medicament. The device comprises a housing, a drive mechanism to be operably engaged with a piston of a cartridge for dispensing of a pre-defined dose of a medicament that is contained in said cartridge. The device further has a cartridge holder as well as a cartridge being displaced in the cartridge holder and being filled with the medicament. Additionally, the drug delivery device comprises a needle assembly as described above, which is adapted to be interconnected with the cartridge holder.

In still another aspect, the invention also provides a method of releasably fastening a needle assembly to a cartridge holder of a drug delivery device. The needle assembly comprises a cup-shaped and elastically deformable receptacle of substantially oval cross section and further comprises a bottom section supporting a needle element adapted to be pierced through a pierceable seal of a cartridge. Fastening of the needle assembly onto the cartridge holder comprises the steps of inserting a distal support of the cartridge holder of substantially circular cross section into the cup-shaped receptacle by displacing the receptacle translationally relative to the cartridge holder in proximal direction until radially inwardly protruding latching elements of the needle assembly establish a positive interlock with an annular rim disposed at the outer circumference of the distal support.

Preferably, releasable fastening of cartridge holder and needle assembly can be attained by exclusively conducting axial relative displacement of cartridge holder and needle assembly. Hence any twisting, screwing or rotational movements of needle assembly and cartridge holder is generally not required for fastening and assembling said components of a drug delivery device. A resilient deformation of the contour and shape of the needle assembly's receptacle is due to the geometric shape of the needle assembly support of the cartridge holder and its fastening means.

In a further preferred embodiment, disassembling of needle assembly and cartridge holder comprises the steps of applying radially inwardly directed pressure on opposite end points of an imaginary long axis of the ovally shaped cup-shaped receptacle for disengaging the positive interlock. Thereafter, needle assembly can be displaced translationally relative to the cartridge holder in axial and distal direction, hence away from the distal end of the cartridge holder.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, a antibody, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(02)25, IsoAsp28] Exendin-4(1-39),
   wherein the group -Lys6-NH2 may be bound to the C-terminus of the Exendin-4 derivative;
or an Exendin-4 derivative of the sequence
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(02)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(02)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(02)25, Asp28] Exendin-4(1-39)-NH2,
   des Pro36, Pro37, Pro38 [Met(O)14, Trp(02)25, Asp28] Exendin-4(1-39)-(Lys)6-NH2,
   H-(Lys)6-des Pro36, Pro37, Pro38 [Met(O)14, Trp(02)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be apparent to those skilled in the pertinent art, that various modifications and variations can be made to the present invention without departing from the spirit and scope of the invention. Further, it is to be noted, that any reference signs used in the appended claims are not to be construed as limiting the scope of the present invention.

Without limitation, the present invention will be explained in greater detail below in connection with preferred embodiments and with reference to the drawings in which:
- Figure 1: schematically illustrates a cartridge holder subassembly with needle assembly attached thereto in cross sectional illustration,
- Figure 2: a simplified cross section through A-A according to Figure 1 in a final assembly configuration and
- Figure 3: the cross section A-A according to Figure 1 during disassembling of needle assembly and cartridge holder.

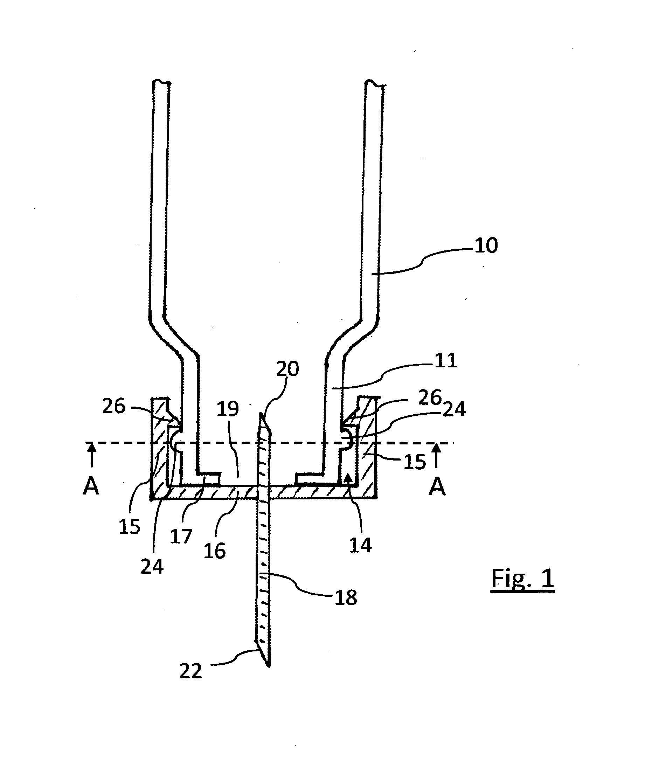

In Figure 1, a cartridge holder subassembly is illustrated in a longitudinal cross section. The needle assembly 12 substantially comprises a cup-shaped receptacle 14 having a bottom section 16 and a substantially cylindrical and circumferential sidewall 15. In the centre of the bottom section 16, a piercing element, typically in form of an injection needle 18 is embedded having a distal tip 22 and a proximal tip 20. By way of the proximal tip 20, the needle 18 penetrates a pierceable septum of a cartridge, which is not explicitly illustrated here.

The cartridge holder 10 forms part of a housing of the not further illustrated drug delivery device. At its lower, distal section, the cartridge holder 10 comprises a neck portion 11, that serves as support for the cup-shaped receptacle 14.

At its distal end section, the cartridge holder 10 comprises a radially inwardly directed circumferential flange portion 17 abutting with the bottom section 16 of the needle assembly 12. Furthermore, radial extension of the flange 17 defines a through opening 19 for the proximal portion 20 of the needle 18.

As can be further seen from Figure 1, needle assembly 12 and cartridge holder 10 are fastened to each other by way of inter-engaging fastening elements 24, 26. As illustrated in Figure 1, the fastening means of the cartridge holder 10 is designed as radially protruding annular rim 24, whereas the corresponding fastening means of the cup-shaped receptacle 14 comprise radially inwardly protruding latching elements 26, that are adapted to form a snap fit with the annular rim 24 of the cartridge holder support 11.

As further illustrated in Figure 1, radially inwardly protruding latching elements 26 comprise a bevelled surface, facilitating establishment of the snap fit as illustrated in Figure 1. Hence, when the needle assembly 12 is simply translationally displaced relative to the cartridge holder 10 in proximal direction, which is upwards in Figure 1, mutual interaction and inter-engagement of the corresponding fastening means 24, 26 will inherently lead to an at least temporary resilient deformation of the cross section of the cup-shaped receptacle 14 until the final assembly configuration is reached as illustrated in Figure 1.

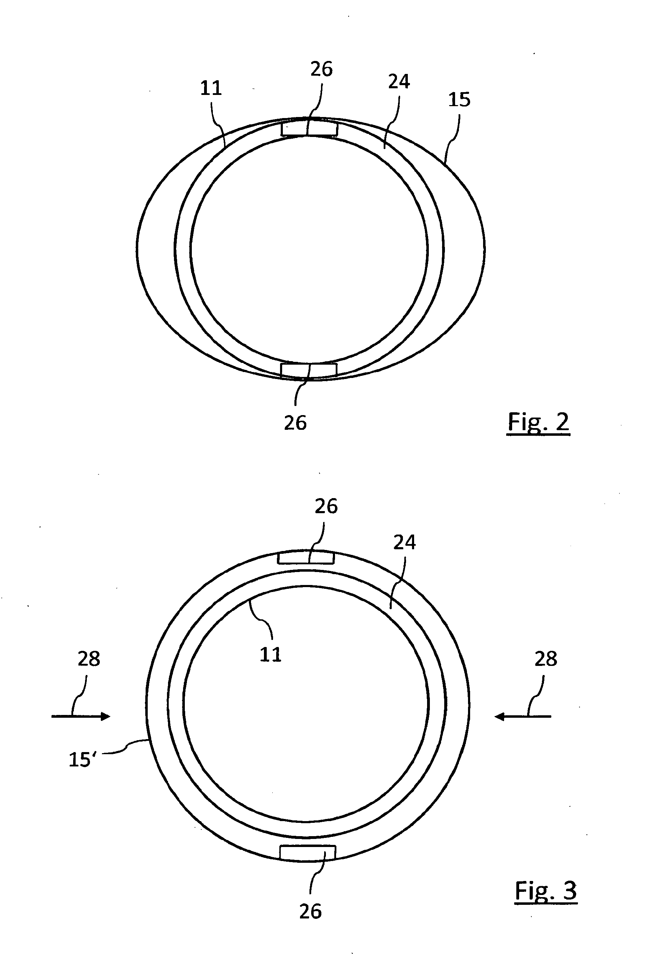

In Figures 2 and 3, a cross section of the cartridge holder 10 along A-A according to Figure 1 is illustrated. The support section 11 is of rather circular symmetric geometry and its outer diameter is approximately in the range of the distance between oppositely disposed latching elements 26 of the receptacle 14 of the needle assembly 12. As illustrated in Figure 2, radially inwardly protruding latching elements 26 of the needle assembly 12 geometrically overlap with the annular rim 24 of the cartridge holder 10. Since the latching elements 26 of the needle assembly 12 also abut in axial direction with the annular rim, the receptacle 14 and the needle assembly 12 are also fixed to the cartridge holder in axial direction.

In the opposite axial direction, mutual abutment of flange portion 17 and bottom section 16 limits any further displacement of the needle assembly 12 in proximal direction relative to the cartridge holder 10.

For releasing the snap fit as illustrated in Figure 1, radially inwardly directed pressure 28 has to be applied to the sidewall 15 of the receptacle 14. Since the receptacle 14 is elastically or resiliently deformable, application of pressure 28 leads to a respective deformation of the cross sectional shape of the needle assembly 12. Hence, the side wall 15 becomes subject to a deformation 15' and features a substantially circular cross section as depicted in Figure 3.

Such a deformation inevitably comes along with an increase of the distance between the latching elements 26. The deformation-induced distance between opposite latching elements 26 becomes larger than the outer diameter of the annular rim 24. Consequently, inter-engagement of the mutually corresponding fastening means 24, 26 of needle assembly 12 and cartridge holder 10 is repealed and the needle assembly 12 is ready to be displaced in axial and distal direction relative to the cartridge holder 10.

Preferably, the receptacle 14 of the needle assembly 10 is manufactured as an injection molded piece and comprises elastomeric or polymeric material comprising a suitable degree of hardness or flexibility allowing for the intended elastic or resilient deformation of the receptacle 14 for the described releasable fastening to the cartridge holder 10.

### List of Reference Numerals

- 10: cartridge holder
- 11: neck portion
- 12: needle assembly
- 14: receptacle
- 15: sidewall
- 16: bottom section
- 17: flange
- 18: needle
- 19: through opening
- 20: tip
- 22: tip
- 24: rim
- 26: latching element
- 28: pressure

## Claims

1. A needle assembly for a drug delivery device comprising:
- a cup-shaped receptacle (14) having a bottom section (16) supporting a needle element (20) defining an axial direction,
- wherein the receptacle (14) comprises fastening means (26) for releasably fastening the needle assembly to the cartridge holder (10), **characterized in that**
- the fastening means (26) is convertible into a release configuration by way of an elastic deformation of the receptacle (14), and
- wherein the cup-shaped receptacle (14) is initially oval in cross section and comprises a resiliently deformable side wall section (15), wherein the fastening means comprise at least two radially inwardly protruding latching elements (26) arranged on opposite endpoints of an imaginary short axis of the oval receptacle (14) and **characterized in that** the needle element is adapted to penetrate a pierceable seal of a cartridge to be disposed in a cartridge holder (10) of the drug delivery device, the fastening means (26) is adapted to form a positive interlock with a corresponding fastening means (24) of the cartridge holder (10) in the axial direction and for axially fastening the receptacle (14) to the cartridge holder (10).

2. The needle assembly according to claim 1, wherein the fastening means (24, 26) is adapted to establish the positive interlock by displacing the cup-shaped receptacle (14) translationally relative to the cartridge holder (10) in a proximal direction.

3. The needle assembly according to any of the preceding claims, wherein the latching elements (26) comprise a bevelled face pointing towards a direction of assembly.

4. The needle assembly according to any one of the preceding claims, wherein the fastening means (26) are convertible into the release configuration by applying radially inwardly directed pressure on opposite end points of an imaginary long axis of the oval receptacle (14).

5. The needle assembly according to any one of the preceding claims, wherein the receptacle (14) transforms to a substantially circular symmetric cross section when converted into its release configuration.

6. A drug delivery device for administering a dose of a medicament, comprising:
- a housing,
- a drive mechanism to be operably engaged with a piston of a cartridge for dispensing of a pre-defined dose of a medicament contained in said cartridge,
- a cartridge holder (10),
- a cartridge disposed in the cartridge holder (10) and being filled with the medicament, and
- a needle assembly (12) according to any one of the preceding claims 1 to 6.

7. A method of releasably fastening a needle assembly (12) according to claim 1 to a cartridge holder (10) of a drug delivery device, wherein fastening of the needle assembly (12) to the cartridge holder (10) comprises the steps of:
- inserting a distal support (11) of the cartridge holder (10) of substantially circular cross section into the cup-shaped receptacle (14) by displacing the receptacle (14) translationally relative to the cartridge holder (10) in proximal direction until radially inwardly protruding latching elements (26) of the needle assembly (12) arranged on opposite endpoints of an imaginary short axis of the oval receptacle (14) form a positive interlock with an annular rim (24) disposed at the outer circumference of the distal support (11).

8. The method according to claim 7, wherein disassembling of needle assembly and cartridge holder (10) comprises the steps of:
- applying radially inwardly directed pressure (28) on opposite end points of an imaginary long axis of the oval shaped cup-shaped receptacle (14) of the needle assembly (12) for disengaging the positive interlock,
- displacing the needle assembly (12) translationally relative to the cartridge holder in distal direction away from the distal end (11) of the cartridge holder (12).

## Patentansprüche

1. Nadelanordnung für eine Medikamenten-Verabreichungsvorrichtung, umfassend:
eine tassenförmige Aufnahme (14) mit einem Bodenabschnitt (16), der ein Nadelelement (20) stützt, das eine axiale Richtung definiert,
wobei die Aufnahme (14) ein Befestigungsmittel (26) umfasst, um die Nadelanordnung lösbar am Kartuschenhalter (10) zu befestigen,
**dadurch gekennzeichnet, dass**
das Befestigungsmittel (26) über eine elastische Deformation der Aufnahme (14) in eine Lösestellung konvertierbar ist, und
wobei die tassenförmige Aufnahme (14) anfänglich einen ovalen Querschnitt hat und einen elastische deformierbaren Seitenwandabschnitt (15) umfasst, wobei die Befestigungsmittel mindestens zwei radial nach innen vorragende Rastelemente (26) umfassen, die an gegenüberliegenden Endpunkten einer gedachten kurzen Achse der ovalen Aufnahme (14) angeordnet sind, und **dadurch gekennzeichnet, dass** das Nadelelement geeignet ist, eine durchstechbare Dichtung einer Kartusche zu durchdringen, die in einem Kartuschenhalter (10) der Medikamenten-Verabreichungsvorrichtung anzuordnen ist, und das Befestigungsmittel (26) geeignet ist, einen Formschluss mit einem entsprechenden Befestigungsmittel (24) des Kartuschenhalters (10) in der axialen Richtung und zur axialen Befestigung der Aufnahme (14) am Kartuschenhalter (10) zu bilden.

2. Nadelanordnung nach Anspruch 1, wobei das Befestigungsmittel (24, 26) geeignet ist, den Formschluss herzustellen, indem es die tassenförmige Aufnahme (14) translational bezüglich des Kartuschenhalters (10) in eine proximale Richtung verschiebt.

3. Nadelanordnung nach einem der vorhergehenden Ansprüche, wobei die Rastelemente (26) eine abgeschrägte Fläche umfassen, die in eine Anordnungsrichtung zeigt.

4. Nadelanordnung nach einem der vorhergehenden Ansprüche, wobei die Befestigungsmittel (26) dadurch in die Lösestellung konvertierbar sind, dass gegenüberliegende Endpunkte einer gedachten langen Achse der ovalen Aufnahme (14) mit radial nach innen gerichtetem Druck beaufschlagt werden.

5. Nadelanordnung nach einem der vorhergehenden Ansprüche, wobei die Aufnahme (14) in einen im Wesentlichen kreisförmigen symmetrischen Querschnitt übergeht, wenn sie in ihre Lösestellung konvertiert wird.

6. Medikamenten-Verabreichungsvorrichtung zur Verabreichung einer Dosis eines Medikaments, umfassend:
- ein Gehäuse,
- einen Antriebsmechanismus, der mit einem Kolben einer Kartusche in Eingriff bringbar ist, um eine vordefinierte Dosis eines in der Kartusche enthaltenen Medikaments abzugeben,
- einen Kartuschenhalter (10),
- eine Kartusche, die im Kartuschenhalter (10) angeordnet ist und mit dem Medikament gefüllt ist, und
- eine Nadelanordnung (12) nach einem der vorhergehenden Ansprüche 1 bis 6.

7. Verfahren zum lösbaren Befestigen einer Nadelanordnung (12) nach Anspruch 1 an einem Kartuschenhalter (10) einer Medikamenten-Verabreichungsvorrichtung, wobei das Befestigen der Nadelanordnung (12) am Kartuschenhalter (10) den folgenden Schritt umfasst:
- Einführen einer distalen Stütze (11) des Kartuschenhalters (10) mit im Wesentlichen kreisförmigem Querschnitt in der tassenförmigen Aufnahme (14), indem die Aufnahme (14) translational bezüglich des Kartuschenhalters (10) so lange in proximale Richtung verschoben wird, bis radial nach innen vorragende Rastelemente (26) der Nadelanordnung (12), die an gegenüberliegenden Endpunkten einer gedachten kurzen Achse der ovalen Aufnahme (14) angeordnet sind, in Formschluss mit einem ringförmigen Rand (24) kommen, der am äußeren Umfang der distalen Stütze (11) angeordnet ist.

8. Verfahren nach Anspruch 7, wobei das Auseinanderbauen der Nadelanordnung und des Kartuschenhalters (10) die folgenden Schritte umfasst:
- Beaufschlagen gegenüberliegender Endpunkte einer gedachten langen Achse der ovalen tassenförmigen Aufnahme (14) der Nadelanordnung (12) mit radial nach innen gerichtetem Druck (28) zum Lösen des Formschlusses, und
- translationales Verschieben der Nadelanordnung (12) bezüglich des Kartuschenhalters in distaler Richtung weg vom distalen Ende (11) des Kartuschenhalters (12).

## Revendications

1. Ensemble d'aiguille pour dispositif d'administration de médicaments, comprenant :
- un réceptacle en forme de coupe (14) ayant une section de fond (16) supportant un élément d'aiguille (20) définissant une direction axiale,
- le réceptacle (14) comprenant un moyen d'attache (26) pour attacher de manière amovible l'ensemble d'aiguille au support de cartouche (10), **caractérisé en ce que**
- le moyen d'attache (26) peut être converti en une configuration de libération par le biais d'une déformation élastique du réceptacle (14),
et
- le réceptacle en forme de coupe (14) étant initialement ovale en section transversale et comprenant une section de paroi latérale déformable élastiquement (15), le moyen d'attache comprenant au moins deux éléments de verrouillage (26) faisant saillie radialement vers l'intérieur, agencés sur des points d'extrémité opposés d'un axe court imaginaire du réceptacle ovale (14) et **caractérisé en ce que** l'élément d'aiguille est prévu pour pénétrer à travers un joint perçable d'une cartouche devant être disposée dans un support de cartouche (10) du dispositif d'administration de médicaments et le moyen d'attache (26) étant prévu pour former un emboîtement positif avec un moyen d'attache correspondant (24) du support de cartouche (10) dans la direction axiale et pour attacher axialement le réceptacle (14) au support de cartouche (10).

2. Ensemble d'aiguille selon la revendication 1, dans lequel le moyen d'attache (24, 26) est prévu pour établir l'emboîtement positif en déplaçant le réceptacle en forme de coupe (14) en translation par rapport au support de cartouche (10) dans une direction proximale.

3. Ensemble d'aiguille selon l'une quelconque des revendications précédentes, dans lequel les éléments de verrouillage (26) comprennent une face biseautée orientée vers une direction d'assemblage.

4. Ensemble d'aiguille selon l'une quelconque des revendications précédentes, dans lequel le moyen d'attache (26) peut être converti en la configuration de libération en appliquant une pression orientée radialement vers l'intérieur sur des points d'extrémité opposés d'un axe long imaginaire du réceptacle ovale (14).

5. Ensemble d'aiguille selon l'une quelconque des revendications précédentes, dans lequel le réceptacle (14) acquiert une forme symétrique substantiellement circulaire en section transversale lorsqu'il est converti en sa configuration de libération.

6. Dispositif d'administration de médicaments pour administrer une dose d'un médicament, comprenant :
- un boîtier,
- un mécanisme d'entraînement destiné à être engagé fonctionnellement avec un piston d'une cartouche destinée à distribuer une dose prédéfinie d'un médicament contenu dans ladite cartouche,
- un support de cartouche (10),
- une cartouche placée dans le support de cartouche (10) et remplie du médicament, et
- un ensemble d'aiguille (12) selon l'une quelconque des revendications 1 à 6.

7. Procédé d'attache amovible d'un ensemble d'aiguille (12) selon la revendication 1, à un support de cartouche (10) d'un dispositif d'administration de médicament, dans lequel l'attache de l'ensemble d'aiguille (12) au support de cartouche (10) comprend l'étape suivants :
- insérer un support distal (11) du support de cartouche (10) de section transversale substantiellement circulaire dans le réceptacle en forme de coupe (14) en déplaçant le réceptacle (14) en translation par rapport au support de cartouche (10) dans la direction proximale jusqu'à ce que des éléments de verrouillage (26) faisant saillie radialement vers l'intérieur de l'ensemble d'aiguille (12), agencés sur les points d'extrémité opposés d'un axe court imaginaire du réceptacle ovale (14) forment un emboîtement positif avec un bord annulaire (24) disposé au niveau de la circonférence extérieure du support distal (11).

8. Procédé selon la revendication 7, dans lequel le démontage de l'ensemble d'aiguille et du support de cartouche (10) comprend les étapes suivantes :
- appliquer une pression orientée radialement vers l'intérieur (28) sur des points d'extrémité opposés d'un axe long imaginaire du réceptacle en forme de coupe de forme ovale (14) de l'ensemble d'aiguille (12) afin de désengager l'emboîtement positif,
- déplacer l'ensemble d'aiguille (12) en translation par rapport au support de cartouche dans la direction distale à l'écart de l'extrémité distale (11) du support de cartouche (12).
